# EUROPÄISCHE PATENTSCHRIFT

(11) **EP 1 962 738 B1**
(45) Veröffentlichungstag und Bekanntmachung des Hinweises auf die Patenterteilung: **01.09.2010**
(21) Anmeldenummer: 06828663.2
(22) Anmeldetag: 13.12.2006
(51) Int. Cl.: A61F 2/68, F16H 3/00

(54) **SCHALTKUPPLUNG FÜR PROTHESEN**
CLUTCH MODULE FOR PROSTHESES
ACCOUPLEMENT DE COMMUTATION DESTINE A DES PROTHESES

(30) Priorität: 23.12.2005 DE 102005062400; 14.02.2006 DE 102006006966
(43) Veröffentlichungstag der Anmeldung: 03.09.2008
(73) Patentinhaber: Otto Bock HealthCare GmbH, 37115 Duderstadt (DE)
(72) Erfinder: PUCHHAMMER, Gregor, 1130 Wien (AT)
(74) Vertreter: Stornebel, Kai
(86) Internationale Anmeldenummer: PCT/DE2006/002226
(87) Internationale Veröffentlichungsnummer: WO 2007/076795

(56) Entgegenhaltungen:
- DE-A1- 2 335 974
- US-A- 3 883 900
- US-A- 5 280 981
- US-A1- 2002 077 708

## Beschreibung

Die Erfindung betrifft eine Schaltkupplung für Prothesen, insbesondere für prothetische Greifgeräte, Armprothesen oder Handprothesen z.B. mit einem Chassis, an dem zumindest eine Fingerprothese gelenkig gelagert ist, die ein Antriebselement, ein Abtriebselement und eine Schalteinrichtung aufweist, die in Abhängigkeit von den an dem Abtriebselement anliegenden Drehmomenten zwischen zwei Getriebestufen umschaltet.

Um in Prothesenkomponenten, Prothesenhänden. Armprothesen oder prothetischen Greifelementen einerseits eine hohe Greifkraft und andererseits eine schnelle Greifbewegung realisieren zu können, sind Schaltgetriebe innerhalb der Prothese vorgesehen, die einerseits eine hohe Übersetzung realisieren können, um Greifelemente schnell in Kontakt mit dem zu greifenden Gegenstand zu bringen, und andererseits auch eine geringe Übersetzung aufweisen, um entsprechend hohe Greifkräfte realisieren zu können. Die Umschaltung zwischen den einzelnen Getriebestufen kann auf unterschiedliche Art und Weise geschehen. In der Regel muss für eine rechte und linke Prothesenhand je ein gesondertes Getriebe bereitgestellt werden. Ebenfalls benötigt man für das Greifen und das Öffnen der Hand separate Getriebe. Gleiches gilt grundsätzlich auch für Armprothesen, prothetische Greifelemente oder andere Prothesen, die mit einem Antrieb ausgestattet sind und zunächst schnell und ohne hohen Krafteinsatz in eine gewünschte Stellung verfahren werden sollen, während eine langsame und kräftige Bewegung erst später erfolgen soll.

Die US 2002/077708 A1 beschreibt einen Antrieb einer Prothesenhand mit einem Antriebselement, einem Abtriebselement und einer Schalteinrichtung in Gestalt eines Zweiganggetriebes mit einem Getriebegehäuse. Mit dem Zweiganggetriebe ist es möglich, bei einer freien Verstellung der Prothesenfinger eine hohe Geschwindigkeit zu realisieren, während bei einem Widerstand über drehmomentgesteuerte Federn eine Umschaltung auf einen zweiten Gang mit einer niedrigen Geschwindigkeit und einem höheren Drehmoment erfolgen kann. Dadurch ist es möglich, hohe Griffkräfte aufzubringen. Über eine Sperreinrichtung werden die Finger in der verriegelten Position gehalten. Die Sperreinrichtung kann gelöst werden. Nach dem Lösen der Sperreinrichtung ist eine Drehmomentübertragung über das Getriebe nicht vorgesehen.

Aufgabe der vorliegenden Erfindung ist es, ein Getriebeelement bereitzustellen, mit dem preiswerter und leichter eine vollfunktionsfähige Prothese oder prothetische Greifeinrichtung ausgestaltet werden kann.

Erfindungsgemäß wird diese Aufgabe durch eine Schaltkupplung mit den Merkmalen des Anspruchs 1 gelöst. Vorteilhafte Ausgestaltungen und Weiterbildungen der Erfindung sind in den Unteransprüchen ausgeführt.

Die erfindungsgemäße Schaltkupplung für Prothesen, insbesondere für prothetische Greifgeräte, Armprothesen oder Handprothesen z.B. mit einem Chassis, an dem zumindest eine Fingerprothese gelenkig gelagert ist, weist ein Antriebselement, ein Abtriebselement und eine Schalteinrichtung auf, die in Abhängigkeit von den an dem Abtriebselement anliegenden Drehmomenten zwischen zwei Getriebestufen umschaltet. Somit wird automatisch eine Anpassung an die erforderliche Verstell- oder Griffkraft ermöglicht, wobei zunächst eine schnelle Verstell- oder Greif- bzw. Öffnungsbewegung realisiert wird, an die sich bei einer Erhöhung des Verstell- oder Greifwiderstandes eine Erhöhung oder Verstell- oder Greifkraft bei gleichzeitiger Verringerung der Verstell- oder Greifgeschwindigkeit anschließt Bei einer Anwendung in einer Handprothese ist die Schaltkupplung auch bei dem Öffnen der Hand einsetzbar. Bei einem Öffnen der Hand erfolgt dieses ebenfalls belastungsabhängig, indem zunächst bei einem hohen Widerstand die geringe Übersetzung mit einer geringen Drehzahl aktiviert wird, um hohe Lösekräfte bzw. Öffnungskräfte bereitzustellen. Ist der Griff gelockert oder haben die Prothesenfinger einen Gegenstand auseinandergedrückt, erhöht sich bei Nachlassen der Gegenkraft die Verstellgeschwindigkeit, so dass eine schnelle Öffnung der Greifelemente möglich ist. Die Umschaltung ist also drehmomentabhängig und drehrichtungsunabhängig; dies ist unabhängig von dem Einsatzgebiet der Schaltkupplung.

Die Erfindung sieht vor, dass die Schalteinrichtung mit Klemmkörpern, insbesondere Klemmrollen in Kontakt steht, die das Antriebselement drehmomentgesteuert mit dem Abtriebselement koppeln. Die Klemmkörper ermöglichen einen verschleißarmen und sicheren Aufbau der Schaltkupplung und gewährleisten eine drehrichtungsunabhängige Kopplung der Kraftübertragungselemente miteinander. Insbesondere kann durch einen symmetrischen Aufbau der Kraftübertragungskomponenten die Drehrichtungsunabhängigkeit einfach realisiert werden.

Die Schalteinrichtung kann zwei Schaltelemente aufweisen, die vorgespannt und verdrehbar zueinander gelagert sind und Schaltstege oder Schaltstifte aufweisen, die drehmomentgesteuert mit den Klemmkörpern in Kontakt bringbar sind. Über den Verdrehwinkel der Schaltelemente zueinander werden bedarfsgesteuert die jeweils zuzuschaltenden Klemmkörpern mit den Kraftübertragungseinrichtungen in Kontakt gebracht und ermöglichen den Antrieb des Abtriebselementes. Dabei sind die Schaltelemente bevorzugt über Federn unter Vorspannung gehalten, so dass bei einer geringen Gegenkraft das Auslösemoment bzw. Ansprechmoment der Schaltkupplung einstellbar ist. Je höher die Vorspannung der Federn ist, desto größer muss die Gegenkraft sein, damit eine ausreichend hohe Verdrehung relativ zueinander stattfindet. Dadurch erhöht sich das Auslösemoment, das an die individuellen Bedürfnisse des Prothesenträgers oder des Anwenders der prothetischen Greifeinrichtung angepasst werden kann. Die Federn sind austauschbar. Gegebenenfalls kann in Abhängigkeit der Verstellbewegung eine unterschiedliche Federrate eingestellt werden, z.B. bei einer Handprothese oder prothetischen Greifeinrichtung für die Öffnungsbewegung eine andere Federrate als für die Schließbewegung, so dass bei unterschiedlichen Gegenkräften unterschiedliche Schaltzeitpunkte oder -momente einstellbar sind, beispielsweise für das Öffnen oder Schließen der Greifelemente oder Prothesenfinger.

Das Antriebselement kann mit einem Getriebe, z.B. einem Planetengetriebe gekoppelt sein, um eine Untersetzung realisieren zu können. Dabei weist das entsprechende Untersetzungsgetriebe einen Kontaktring auf, mit dem die Klemmkörper zur Übertragung eines hohen Drehmomentes in Kontakt stehen.

Bevorzugt sind die Klemmkörper oder -rollen auf einer gemeinsamen Mitnehmerscheibe angeordnet, um den Bauraum möglichst gering zu halten. Die Mitnehmerscheibe weist einen Rahmen auf, in dem die Klemmkörper für eine hohe Übersetzung angeordnet sind. Außerhalb des Rahmens sind die Klemmkörper für die Übertragung eines hohen Momentes bei einer niedrigen Übersetzung angeordnet, die in Kontakt mit einem Kontaktring des Unterseizungsgetriebes stehen. Die Anwendung von Klemmkörpern sowohl für die Übersetzung mit einem hohen Moment bei geringer Drehzahl als auch für die Übersetzung mit einem geringen Moment bei hoher Drehzahl hat den Vorteil, dass ein gleitender Übergang zwischen den einzelnen Getriebestufen realisierbar ist, ohne dass ein merkbarer Schaltvorgang innerhalb der Handprothese spürbar ist.

Auf der Mitnehmerscheibe sind Zahnräder gelagert, die in Eingriff mit einem Schaltring stehen und diesen relativ zu einer Schaltscheibe und einem zweiten Schaltelement verdrehen. Über diese Verdrehung wird winkelabhängig eine Ablaufsteuerung des Ineingrifftretens oder Außereingriffstellens der Klemmkörper oder -rollen zu den jeweiligen Kraftübertragungskomponenten bewirkt. Nachfolgend wird ein Ausführungsbeispiel anhand der beigefügten Figuren näher erläutert. Es zeigen:
- Figur 1: - eine Schaltkupplung in Explosionsdarstellung;
- Figur 2: - eine Schnittdarstellung der Schaltkupplung im Kraftgang;
- Figur 3: - eine Schnittdarstellung gemäß Figur 2 in einer anderen Ebene;
- Figur 4: - eine zusammengebaute Schaltkupplung.

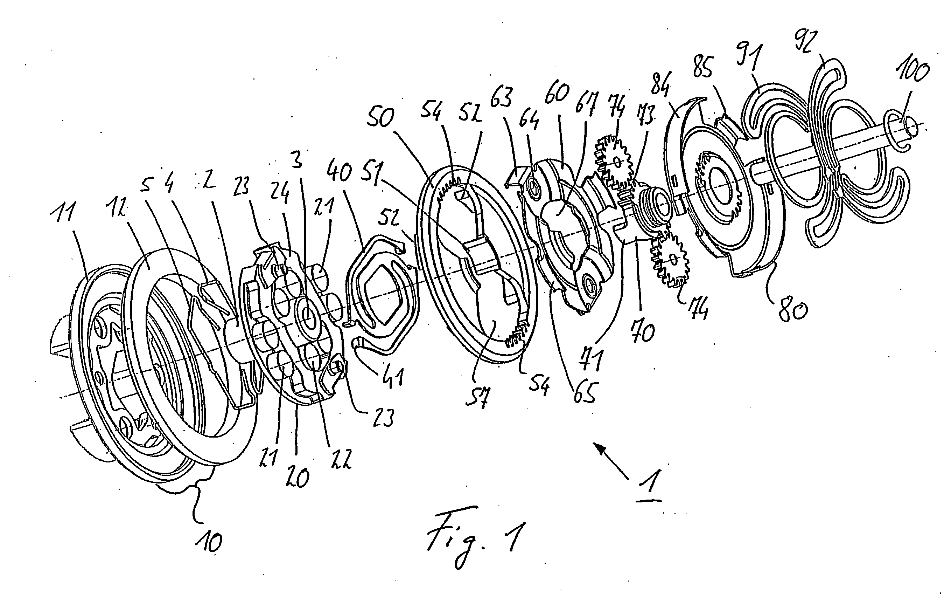

In der Figur 1 ist eine Schaltkupplung 1 in einer Explosionsdarstellung gezeigt. Die Schaltkupplung 1 dient zum drehmomentgesteuerten Umschalten zwischen einem Schnellgang und einem Kraftgang und weist eine Antriebswelle 2 auf, auf der eine Mitnehmerscheibe 20 fliegend gelagert ist. Diese Mitnehmerscheibe 20 ist in einem Planetenträgergehäuse 10 drehbar gelagert. Innerhalb des Planetenträgergehäuses 10 befindet sich ein Planetengetriebe, ein Harmonic-Drive-Getriebe oder ein anderes hoch untersetztes Getriebe, das aus Übersichtlichkeitsgründen nicht dargestellt ist. Das Planetenträgergehäuse 10 weist einem Kontaktring 12 auf, auf dessen Innenumfang eine Lauffläche ausgebildet ist. Aus Stabilitätsgründen ist dieser Kontaktring 12 massiv, bevorzugt aus Stahl ausgebildet. Alternativ kann die Lauffläche des Kontaktringes 12 aus einem gehärteten Stahlring bestehen und die Lauffläche ausbilden. Unterhalb des Kontaktringes 12 ist ein Kunststoffgehäuse 11 angeformt, das aus Gewichtsgründen bevorzugt aus Kunststoff ausgebildet ist. Der Kontaktring 12 und das Gehäuse 11 können aneinander befestigt oder zusammen im Zweikomponenten-Spritzgussverfahren hergestellt sein.

Die Antriebswelle 2 ist zylindrisch ausgebildet und weist eine Bohrung 3 auf, in der ein Montagestift 100 aufgenommen sein kann, um den sich die weiteren Elemente der Schaltkupplung 1 herum gruppieren. Die Antriebswelle 2 treibt ständig das nicht dargestellt Planetengetriebe und damit das Planetenträgergehäuse 10 und den Kontaktring 12 an, sofern ein nicht dargestellter Motor aktiviert ist. Der Kontaktring 12 läuft also bei eingeschaltetem Antrieb ständig um die Trägerscheibe 20 um.

Auf der Trägerscheibe 20 ist ein Rahmen 24 ausgebildet, der eine innere Aussparung aufweist, in der insgesamt vier Klemmkörper in Gestalt von Klemmrollen 22 angeordnet sind. Weiterhin weist der Rahmen 24 äußere Aussparungen auf, die äußere Klemmkörper in gestalt von Klemmrollen 21 aufnehmen können. Ebenfalls sind auf dem Rahmen 24 Stifte 23 ausgebildet, die als Achsen für Zahnräder 74 dienen, deren Funktion später erläutert werden wird. Zwischen der Trägerscheibe 20 und dem Planetenträgergehäuse 10 sind zwei Federn 4, 5 angeordnet, die die Klemmrollen 21, 22 in ihren gewünschten Positionen halten. Zum gleichen Zweck ist auf der dem Planetenträgergehäuse 10 abgewandten Seite der Mitnehmerscheibe 20 eine S-förmig gestaltete Feder 40 angeordnet, die nach unten ragende Stege 41 aufweist, die mit den äußeren Klemmrollen 21 in Kontakt stehen und diese gegen Schaltstege oder -stifte 52 eines oberhalb angeordneten Schaltringes 50 drücken. Dadurch wird eine spiellose Schaltung der äußeren Klemmrollen 21 bewirkt.

Oberhalb der Federscheibe 40 ist der Schaltring 50 angeordnet, der vier nach unten ragende, radial außen angeordnete Schaltstege 52 oder Schaltstifte aufweist, die zur drehmomentgesteuerten Aktivierung der äußeren Klemmrollen 21 dienen. Weiterhin weist der Schaltring 50 radial innen liegende Schaltstege 51 oder Schaltstifte auf, die zur Aktivierung oder Deaktivierung der inneren Klemmrollen 12 dienen. Innerhalb des Schaltringes 50 sind Ausnehmungen 57 für die Zahnräder 74 ausgebildet, die in eine partielle Innenverzahnung 54 eingreifen.

Auf dem Schaltring 50 ist eine Schaltscheibe 60 angeordnet, die nach unten ragende Kontaktstege 63 aufweist, die um entsprechende Ausnehmungen der Mitnehmerscheibe 20 herumgelegt werden, beispielsweise verpresst oder verclipst, und somit ein Drehmoment übertragen können. Die Schaltscheibe 60 weist Durchgangslöcher 64 zum Durchgang der Stifte 23 auf. Ebenfalls sind an der Schaltscheibe 60, die als Blechteil ausgebildet sein kann, nach oben gebogene Aufnahmestege 65 ausgebildet, in die Federn 91, 92 eingehängt werden können. Innerhalb der Schaltscheibe 60 ist eine Ausnehmung 67 ausgebildet, in der ein Zahnradelement 70 mit einer Teilaußenverzahnung 73 und nach unten ragenden Schaltstegen 71 oder Schaltstiften drehbar gelagert ist. Diese Schaltstege 71 liegen radial innen zu den Schaltstegen 51 des Schaltringes 50 und aktuieren diagonal einander gegenüberliegende, innere Klemmrollen 22.

Oberhalb des Zahnradelementes 70 ist eine Tellerscheibe 80 angeordnet, die einen äußeren, teilweise umlaufenden Steg 84 aufweist, in dem die Drehfedern 91, 92 gelagert sind. Ebenfalls sind Federaufnahmestege 85 nach oben gebogen ausgebildet, die radial innen zu den Federstegen 65 an der Schaltscheibe 60 angeordnet sind.

Die Federn 91, 92 sind vorgespannt und greifen in die Federstege 85, 65 der Tellerscheibe 80 und der Schaltscheibe 60 ein und halten diese zueinander unter Vorspannung.

Die Funktionsweise der Schaltkupplung 1 wird anhand der Anwendung in einer Handprothese erläutert, grundsätzlich kann die Schaltkupplung auch in Armprothesen, prothetischen Greifelementen oder anderen Prothesen mit einem Antrieb eingesetzt werden. Bei Handprothesen oder Greifelementen ist es wichtig, zunächst einen Schnellgang für eine schnelle Verstellung beispielsweise der Prothesenfinger zu ermöglichen, um einen schnellen Kontakt der Prothesenfinger oder Greifelemente mit dem zu ergreifenden Gegenstand zu ermöglichen. Sobald ein erster Kontakt vorhanden ist, ist eine gesteuerte Krafterhöhung wünschenswert, um den gegriffenen Gegenstand auch dauerhaft halten zu können. Dazu ist es notwendig, höhere Momente aufzubringen. Die Momentenerhöhung erfolgt über das nicht dargestellte Planetengetriebe auf Kosten der Verstellgeschwindigkeit.

Im sogenannten Schnellgang, bei dem also eine schnelle Auf- bzw. Zu-Bewegung der Greifelemente gewünscht ist, wird über einen nicht dargestellten Antrieb die Antriebswelle 2 in Rotation versetzt. Die innen liegenden Klemmrollen 22 liegen dabei in der Ausgangsstellung, also bei nicht aktiviertem Antrieb, stets leicht an dem Rahmen 24 an; dies wird durch die Federn 4, 5 gewährleistet. Wird der Antrieb angeschaltet, treten die jeweils diagonal einander gegenüberliegenden inneren Klemmrollen 22 in einen Drehmoment übertragenden Kontakt mit dem Rahmen und verklemmen sich, so dass sich ohne eine Verzögerung das Drehmoment über die Klemmwirkung von der Antriebswelle 2 und den Rahmen 24 auf die Mitnehmerscheibe 20 übertragen. Die Verzögerungsfreiheit wird über die Federvorspannung der Federn 4, 5 gewährleistet. Über die Stifte 23 wird dann die Schaltscheibe 65 und darüber die Tellerscheibe 80 mit dem Moment beaufschlagt, so dass dieses von der Tellerscheibe 80, das gleichzeitig das Abtriebselement ist, an entsprechende Kraftübertragungseinrichtungen abgegeben wird.

In dieser Schnellgangsstellung werden die Tellerscheibe und die Schaltscheibe 65 über die vorgespannten Federn 91, 92 im Wesentlichen in der Ausgangsstellung gehalten, so dass keine Relativverdrehung zwischen der Tellerscheibe 80 und der Schaltscheibe 65 stattfindet. Es wird also eine 1:1-Übersetzung von der Antriebswelle 2 zu dem Abtriebselement in Gestalt der Tellerscheibe 80 ausgeführt. Die Kraftübertragung erfolgt dabei über die inneren Klemmrollen 22, die an die innen umlaufende Antriebswelle 2 und die Innenseite des Rahmens 24 gedrückt werden. Durch die schnelle Drehung kann eine schnelle Bewegung ermöglicht werden.

Wie in der Figur 1 deutlich zu erkennen ist, sind vier innen liegende Klemmrollen 22 innerhalb des Rahmens 24 ausgebildet. Je nach Drehrichtung stehen zwei innere Klemmrollen 22, die einander diagonal gegenüber liegen, in Kraftübertragungseingriff, so dass eine symmetrische Momentenübertragung stattfinden kann. Aufgrund der symmetrischen Anordnung ist die Schaltkupplung sowohl im Rechtslauf als auch im Linkslauf betreibbar, so dass drehrichtungsunabhängig im Schnellgang die entsprechende Bewegung ausgeführt werden kann.

Treten nun die nicht dargestellten Greifelemente oder Prothesenfinger in Kontakt mit dem zu greifenden Gegenstand, ergibt sich ein Widerstand gegen die Drehbewegung des Abtriebselements 80. Sofern der Widerstand einen Grenzwert nicht überschreitet, bleibt die Kraftübertragung durch die innen liegenden Klemmrollen 22 gewährleistet. Überschreitet der Widerstand ein Moment, das durch die Vorspannung der beiden Torsionsfedern 91, 92 vorgegeben ist, verdrehen sich die Tellerscheibe 80 und die Schaltscheibe 60 relativ zueinander. Dann setzt die Umschaltung zwischen dem Schnellgang und dem sogenannten Kraftgang ein, bei der die Verstellung verlangsamt, das aufbringbare Moment jedoch erhöht wird. Diese Umschaltung findet in Gestalt einer mechanischen Ablaufsteuerung statt, bei der drehrichtungsunabhängig und momentengesteuert die innen liegenden Klemmrollen 22 langsam außer Kontakt mit der Antriebswelle 2 gebracht werden, während gleichzeitig die außen liegenden Klemmrollen 21, die im Schnellgang zur Reibungsvermeidung außer Kontakt mit dem Kontaktring 12 stehen, in Eingriff mit dem Kontaktring 12 gebracht werden, um eine Momentenübertragung durchzuführen.

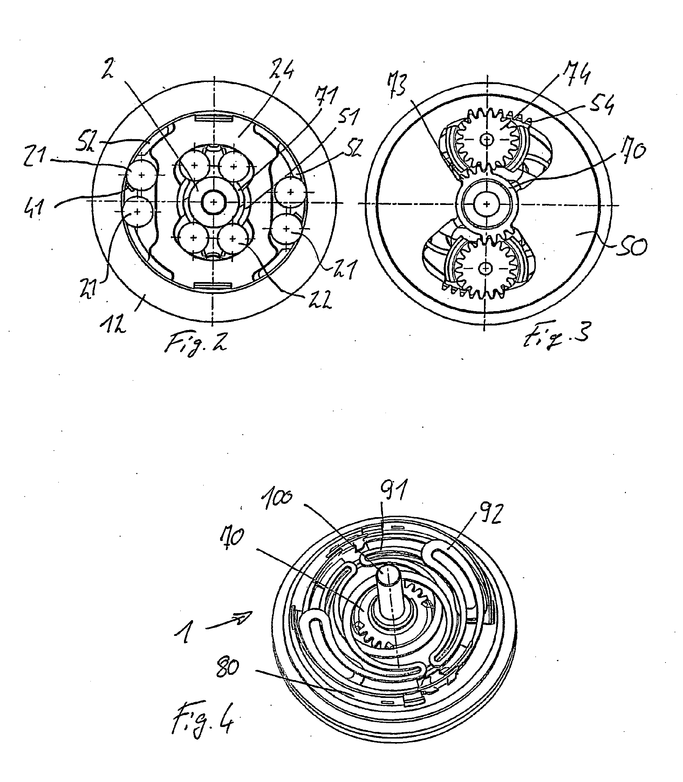

Übersteigt das Widerstandsmoment die Vorspannung der Drehfedern 91, 92, verdrehen sich die Tellerscheibe 80 und die Schaltscheibe 65 relativ zueinander. Über die Zahnräder 74 erfolgt eine Verdrehung des Schaltringes 50 und relativ dazu des Zahnradelementes 70. Dadurch werden die insgesamt acht Schaltstege 51, 52, 71 relativ zueinander verdreht. Diese Verdrehung ist gut in der Figur 2 zu erkennen, auf die später noch eingegangen werden wird.

Die innen liegenden Schaltstege 51, 71 treten in Eingriff mit den innen liegenden Klemmrollen 22 und bewegen diese innerhalb des Rahmens 24 leicht radial nach außen, um diese außer Eingriff mit der Antriebswelle 2 zu bringen. Gleichzeitig verlagern die außen liegenden Schaltstege 52 die diagonal zueinander liegenden äußeren Klemmrollen 21 der Gestalt, dass ein diagonal gegenüberliegendes Klemmrollenpaar drehrichtungsabhängig in Eingriff mit dem Kontaktring 12 tritt.

In der Figur 2 ist eine solche Schaltstellung gezeigt. Die inneren Schaltstege 71, 51 sind gegeneinander verdreht, wobei die Schaltstege 51 des Schaltringes 50 im Uhrzeigersinn und die Schaltstege 71 des Zahnradelementes 70 entgegen dem Uhrzeigersinn verdreht sind. Die äußeren Schaltstege 52 des Schaltringes 50 sind dementsprechend im Uhrzeigersinn verdreht und bewirken, dass die zunächst außer Kontakt befindlichen äußeren Klemmrollen 21, vorliegend die Klemmrollen links oben und rechts unten, in Kontakt mit dem Kontaktring 12 treten. Diese Klemmrollen übertragen dann die Momente von dem sich im Uhrzeigersinn drehenden Kontaktring 12 über die Klemmrollen 21 auf den Rahmen 24 und von dort über die Schaltscheibe 65 und die Tellerscheibe 80 auf das entsprechend anzutreibende, nicht dargestellte Kraftüberträgungselement. Die nach unten gebogenen Stege 41 der Feder 40 sind hier teilweise zu erkennen.

Die nicht an der Kraftübertragung beteiligten Klemmrollen 21 werden über die korrespondierenden Schaltstege 52 außer Eingriff gebracht und laufen frei mit. Sobald das zu übertragende Moment reduziert wird, was sich anhand einer reduzierten Gegenkraft bemerkbar macht, wird die Relativverdrehung zwischen der Schaltscheibe 60 und der Tellerscheibe 80 aufgrund der Federvorspannung der Torsionsfedern 91, 92 rückgängig gemacht. Die außen liegenden Schaltstege 52 verdrehen sich entgegen dem Uhrzeigersinn, wobei sie entgegen der Federwirkung der Feder 40 über die Stege 41 die zunächst in Kontakt befindlichen Klemmrollen 21 außer Eingriff bringen. Gleichzeitig werden die inneren Schaltstege 71, 51 in ihre Ausgangsstellung verdreht, so dass die inneren Klemmrollen 22 wieder in Eingriff treten können und einen Schnellgang bewirken. Aufgrund der beengten Platzverhältnisse macht die Figur 2 nicht deutlich, dass in dem Kraftgang die inneren Klemmrollen 22 außer Eingriff gebracht sind. Hierzu werden auch nur 10tel Millimeter benötigt, die zeichnerisch schwer darstellbar sind.

In der Figur 3 ist ein Schnitt in der Ebene des Schaltringes 50 gezeigt, aus der zu erkennen ist, dass der Schaltring 50 in Uhrzeigerrichtung verdreht wurde. Die Zahnräder 74 befinden sich an dem jeweiligen Ende des Teilinnengewindes 54. Ebenso ist das Teilaußengewinde 73 entsprechend entgegen dem Uhrzeigersinn verdreht. Dieser Winkelversatz führt zur oben beschriebenen Ablaufsteuerung für das Inein-griffbringen oder Außereingriffbringen der Klemmrollen 21, 22. Die Winkelverstellung zwischen dem Abtriebselement bzw. der Tellerscheibe 80 und der Schaltscheibe 60 erfolgt momentenabhängig. Das Schaltmoment kann durch eine Anpassung der Federwerte der Drehfedern 91, 92 eingestellt werden. Gleiches gilt für das Ansprechmoment, also den Beginn des Schaltvorganges.

Das Ineingrifftreten der vier äußeren Klemmrollen 21 und das Außereingriffbringen der vier inneren Klemmrollen 22 ist ein fließender Übergang, so dass teilweise ein überbestimmtes System der Kraftübertragung stattfindet. Zunächst treten die äußeren Klemmrollen 21 leicht in Kontakt mit dem Kontaktring 12, während die inneren Klemmrollen 22 noch in Kontakt mit der Antriebswelle 2 stehen. Dadurch werden ein sanfter Schaltübergang und eine entsprechende Momentenerhöhung bewirkt, so dass kein Schaltschock oder ein harter Übergang zwischen den beiden Schaltstufen entsteht.

In der Figur 4 ist die Schaltkupplung 1 in montiertem Zustand dargestellt. Die Schaltkupplung 1 weist einen sehr kompakten Aufbau auf, da sämtliche Bauteile eine geringe axiale Erstreckung aufweisen. Der drehsymmetrische Aufbau der Schaltkupplung ermöglicht ein drehrichtungsunabhängiges Einsetzen, so dass für eine Handprothese sowohl ein schnelles Öffnen als auch ein schnelles Schließen sowie ein kräftiges Halten bzw. hohes Lösemoment bereitgestellt werden können.

Der Montagestift 100 erleichtert das Zusammensetzen der Schaltkupplung 1, kann jedoch auch entfallen.

## Patentansprüche

1. Schaltkupplung für Prothesen, insbesondere für prothetische Greifgeräte, Armprothesen oder Handprothesen, mit einem Chassis, an dem zumindest eine Fingerprothese gelenkig gelagert ist, die ein Antriebselement (2), ein Abtriebselement (80) und eine Schalteinrichtung (50, 60, 70) aufweist, die in Abhängigkeit von den an dem Abtriebselement (80) anliegenden Drehmomenten zwischen zwei Getriebestufen umschaltet, wobei die Schalteinrichtung (50, 60, 70) unabhängig von der Drehrichtung des Antriebselementes (2) zwischen den Getriebestufen mit unterschiedlicher Übersetzung hin und her schaltet, **dadurch gekennzeichnet, dass** die Schalteinrichtung (50, 60, 70) mit Klemmkörpern (21, 22), insbesondere Klemmrollen, in Kontakt steht, die das Antriebselement (2) drehmomentgesteuert mit dem Abtriebselement (80) koppeln.

2. Schaltkupplung nach Anspruch 1, **dadurch gekennzeichnet, dass** das Abtriebselement (80) bei einem geringen Moment mit einer hohen Übersetzung und bei hohen Momenten mit einer geringen Übersetzung angetrieben ist.

3. Schaltkupplung nach einem der Ansprüche 1 oder 2, **dadurch gekennzeichnet, dass** die Schalteinrichtung (50, 60, 70) zwei Schaltelemente (50, 70) aufweist, die vorgespannt drehbar zueinander gelagert sind und Schaltstege (51, 52, 71) oder Schaltstifte aufweisen, die momentengesteuert mit den Klemmkörpern (21, 22) in Kontakt bringbar sind.

4. Schaltkupplung nach Anspruch 3, **dadurch gekennzeichnet, dass** die Schaltelemente (50, 70) über Federn (91, 92) unter Vorspannung gehalten sind.

5. Schaltkupplung nach einem der voranstehenden Ansprüche, **dadurch gekennzeichnet, dass** das Antriebselement (2) mit einem Getriebe, insbesondere einem Planetengetriebe gekoppelt ist, das einen Kontaktring (12) aufweist, mit dem die Klemmkörper (21) zur Übertragung eines hohen Momentes in Kontakt stehen.

6. Schaltkupplung nach einem der voranstehenden Ansprüche, **dadurch gekennzeichnet, dass** die Klemmkörper (21, 22) auf einer gemeinsamen Mitnehmerscheibe (20) angeordnet sind.

7. Schaltkupplung nach Anspruch 6, **dadurch gekennzeichnet, dass** die Mitnehmerscheibe (20) einen Rahmen (24) aufweist, an dem die Klemmkörper (21) für eine niedrige Übersetzung angeordnet sind.

8. Schaltkupplung nach Anspruch 6, **dadurch gekennzeichnet, dass** die Mitnehmerscheibe (20) einen Rahmen (24) aufweist, in dem die Klemmkörper (22) für die hohe Übersetzung angeordnet sind.

9. Schaltkupplung nach einem der Ansprüche 6 bis 8, **dadurch gekennzeichnet, dass** auf der Mitnehmerscheibe (20) Zahnräder (74) gelagert sind, die in Eingriff mit einem Schaltring (50) stehen und diesen relativ zu einer Schaltscheibe (60) und einem Schaltelement (70) verdrehen.

## Claims

1. A clutch module for prostheses, in particular for prosthetic grippers, arm prostheses or hand prostheses, comprising a chassis, to which at least one finger prosthesis is articulated, and having a drive element (2), a driven element (80) and a shift device (50, 60, 70), which shifts between two gear stages depending on the torques applied to the driven element (80), wherein the shift device (50, 60, 70) shifts back and forth between the gear stages with different increases in speed independently of the direction of rotation of the drive element (2), **characterized** that the shift device {50, 60, 70) is in contact with clamping bodies (21, 22), in particular clamping rollers, which couple the drive element (2) to the driven element {80) in a torque-controlled manner.

2. The clutch module as claimed in claim 1, **characterized in that** the driven element (80) is driven for a low torque with a high increase in speed and for high torques with a low increase in speed.

3. The clutch module as claimed in claim 1 or 2, **characterized in that** the shift device (50, 60, 70) has two switching elements (50, 70), which are supported such that they are prestressed and rotatable in relation to each other and have switching webs (51, 52, 71} or switching pins, which can be bought into contact with the clamping bodies (21, 22) in a torque-controlled manner.

4. The clutch module as claimed in claim 3, **characterized in that** the switching elements (50, 70) are kept under prestress by means of springs (91,92).

5. The clutch module as claimed in one of the preceding claims, **characterized in that** the drive element (2) is coupled to a gear mechanism, in particular a planetary gear mechanism, which has a contact ring (12), with which the clamping bodies (21) for the transmission of a high torque are in contact.

6. The clutch module as claimed in one of the preceding claims, **characterized in that** the clamping bodies (21, 22) are arranged on a common driver disk (20).

7. The clutch module as claimed in claim 6, **characterized in that** the driver disk (20) has a frame (24), on which the clamping bodies (21) for a low increase in speed are arranged.

8. The clutch module as claimed in claim 6, **characterized in that** the driver disk (20) has a frame (24), in which the clamping bodies (22) for the high increase in speed are arranged.

9. The clutch module as claimed in one of claims 6 to 8, **characterized in that** gearwheels (74) which are in engagement with a switching ring (50) and turn the latter in relation to a switching disk (60) and a switching element (70) are mounted on the driver disk (20).

## Revendications

1. Accouplement à changement de vitesse destiné à des prothèses, en particulier pour appareils de préhension prothétiques, prothèses de bras ou prothèses de main, comprenant un châssis auquel est montée articulée au moins une prothèse de doigt, qui présente un élément menant (2), un élément mené (80) et un dispositif de changement (50, 60, 70) qui change entre deux étages de transmission en fonction des moments appliqués à l'élément entraîné (80), le dispositif de changement (50, 60, 70) changeant en va et vient entre les étages de transmission ayant un rapport de transmission différent, et ce indépendamment du sens de rotation de l'élément menant (2), **caractérisé en ce que** le dispositif de changement (50, 60, 70) est en contact avec des corps de serrage (21, 22), en particulier des galets de serrage, qui couplent de façon pilotée par le moment l'élément menant (2) avec l'élément mené (80).

2. Accouplement à changement de vitesse selon la revendication 1, **caractérisé en ce que** l'élément mené (80) est entraîné avec un grand rapport de transmission lorsque le moment est faible et avec un petit rapport de transmission lorsque le moment est élevé.

3. Accouplement à changement de vitesse selon la revendication 1 ou 2, **caractérisé en ce que** le dispositif de changement (50, 60, 70) présente deux éléments de changement (50, 70) qui sont montés rotatifs l'un par rapport à l'autre sous précontrainte et présentent des talons de changement (51, 52, 71) ou des doigts de changement qui peuvent être amenés, de façon pilotée par le moment, en contact avec les corps de serrage (21, 22).

4. Accouplement à changement de vitesse selon la revendication 3, **caractérisé en ce que** les éléments de changement (50, 70) sont maintenus sous précontrainte par des ressorts (91, 92).

5. Accouplement à changement de vitesse selon l'une des revendications précédentes, **caractérisé en ce que** l'élément menant (2) est couplé à une transmission, en particulier une transmission planétaire, qui présente une couronne de contact (12) avec laquelle les corps de serrage (21) sont en contact pour la transmission d'un moment élevé.

6. Accouplement à changement de vitesse selon l'une des revendications précédentes, **caractérisé en ce que** les corps de serrage (21, 22) sont agencés sur un disque d'entraînement (20) commun.

7. Accouplement à changement de vitesse selon la revendication 6, **caractérisé en ce que** le disque d'entraînement (20) présente un cadre (24) contre lequel sont placés les corps de serrage (21) pour un petit rapport de transmission.

8. Accouplement à changement de vitesse selon la revendication 6, **caractérisé en ce que** le disque d'entraînement (20) présente un cadre (24) dans lequel sont placés les corps de serrage (22) pour le grand rapport de transmission.

9. Accouplement à changement de vitesse selon l'une des revendications 6 à 8, **caractérisé en ce que** sur le disque d'entraînement (20) sont montés des pignons (74) qui engrènent avec une couronne de changement (50) et la font tourner par rapport à un disque de changement (60) et un élément de changement (70).
